(19) Europäisches Patentamt
European Patent Office
Office européen des brevets

(11) Veröffentlichungsnummer: **0 324 154 B1**

(12) **EUROPÄISCHE PATENTSCHRIFT**

(45) Veröffentlichungstag der Patentschrift: **17.03.93**

(51) Int. Cl.[5]: **C07C 211/18**, C07F 15/00, A61K 31/28

(21) Anmeldenummer: **88121568.5**

(22) Anmeldetag: **23.12.88**

(54) **1,2-Bis(aminomethyl)cyclobutan-Platin-Komplexe.**

(30) Priorität: **09.01.88 DE 3800415**

(43) Veröffentlichungstag der Anmeldung:
**19.07.89 Patentblatt 89/29**

(45) Bekanntmachung des Hinweises auf die Patenterteilung:
**17.03.93 Patentblatt 93/11**

(84) Benannte Vertragsstaaten:
**AT BE CH DE ES FR GB GR IT LI LU NL SE**

(56) Entgegenhaltungen:
**EP-A- 0 166 366** **EP-A- 0 185 225**
**EP-A- 0 237 450** **DE-A- 1 618 050**
**GB-A- 2 024 823** **GB-A- 2 066 819**

**JOURNAL OF CLINICAL HERMATOLOGY AND ONCOLOGY, Band 7, Nr. 1, 1977, S. 231-241; L. M. HALL et al: "Analogs of sulfato 1,2-diaminocyclohexane platinum(II)(SHP). II. Modifications other than leaving ligand"**

(73) Patentinhaber: **ASTA Medica Aktiengesellschaft**
**Weismüllerstrasse 45**
**W-6000 Frankturt am Main 1(DE)**

(72) Erfinder: **Schumacher, Wolfgang, Dr.**
**Lörracher Strasse 14**
**W-6800 Mannheim 61(DE)**
Erfinder: **Respondek, Johannes, Dr.**
**Friedrichstrasse 5c**
**W-6450 Hanau(DE)**
Erfinder: **Engel, Jürgen, Dr.**
**Erlenweg 3**
**W-8755 Alzenau(DE)**
Erfinder: **Pohl, Jörg, Dr.**
**Eisweg 30**
**W-4802 Halle(DE)**
Erfinder: **Voegeli, Rainer, Dr.**
**Bretonische Strasse 67**
**W-4800 Bielefeld 12(DE)**
Erfinder: **Hilgard, Peter, Dr.**
**Kronenstrasse 11a**
**W-4800 Bielefeld 1(DE)**

**EP 0 324 154 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Durch die britische Patentanmeldung 2 024 823 sind beispielsweise Platin-Komplexe des 1,1-Bis-(aminomethyl)cyclobutans bekannt. Diese Verbindungen werden zur Behandlung von Krebskrankeiten empfohlen.

Die erfindungsgemäßen Verbindungen besitzen eine gute Antitumorwirksamkeit (beispielsweise in vitro am AH 135-Tumor, B 16-Melanom, Colon 115; in vivo beispielsweise am P 388 Leukämie der Maus). Darüber hinaus weisen die erfindungsgemäßen Verbindungen nur eine geringe Toxizität auf, insbesondere haben sie keine kumulative Toxizität und besitzen keine Nephrotoxizität. Weiterhin ist die Knochenmarkstoxizität gering, und die gefürchtete Thrombozytopenie tritt nicht auf.
Außerdem sind die erfindungsgemäßen Verbindungen überraschend gut wasserlöslich.

Die folgenden Angaben betreffen bevorzugte Ausgestaltungen der Erfindung:
Die vorkommenden $C_1$-$C_6$-Alkylgruppen, Alkoxygruppen und die $C_2$-$C_6$-Alkanoyloxygruppen können gerade oder verzweigt sein. Die Alkyl- beziehungsweise Alkoxygruppen bestehen vorzugsweise aus 1 bis 4 C-Atomen, die Alkanoyloxygruppen vorzugsweise aus 2 bis 4 C-Atomen. Dasselbe gilt auch, wenn $C_1$-$C_6$-Alkylgruppen Bestandteil anderer funktioneller Gruppen sind. Als Alkanoyloxygruppe kommt insbesondere die Acetoxygruppe in Frage. Als Halogensubstituenten kommen insbesondere Brom, Chlor und/oder Fluor in Frage. Im Falle der Phenyl-$C_1$-$C_6$-alkylgruppebesteht der Alkylteil vorzugsweise aus einem, zwei oder drei C-Atomen vorzugsweise handelt es sich um die Benzylgruppe oder die 1-Phenylethylgruppe, wobei der Phenylteil gegenbenenfalls jeweils wie angegeben substituiert sein kann.
Vorhandene Phenylreste können ein-, zwei- oder dreifach durch die angegebenen Reste, die gleich oder verschieden sein können, substituiert sein, zum Beispiel kann ein solcher Phenylrest 1 oder 2 Halogenatome (wie Chlor), vorzugsweise in 2- und/oder 6-Stellung enthalten sowie zusätzlich eine Hydroxygruppe (vorzugsweise in 4-Stellung).

Besonders günstige Wirkung besitzen solche Verbindungen der Formel $I'$ oder $I''$, worin sämtliche Reste $R_1$ bis $R_6$ Wasserstoff bedeuten oder worin die Reste $R_1$ bis $R_4$ Wasserstoff sind und einer oder beide Reste $R_5$ und/oder $R_6$ eine $C_1$-$C_4$-Alkylgruppe insbesondere eine Methylgruppe darstellen.

Die Reste X, die gleich oder verschieden sein können, stellen die bekannten und üblichen physiologisch verträglichen und pharmazeutisch verwendbaren Anionen ein- oder mehrwertiger Säuren oder auch das Hydroxyanion $OH^-$) dar. Falls solche Säuren asymmetrische C-Atome haben, können diese als Racemate, als optisch reine Formen oder in Form der entsprechenden Diastereomere vorliegen. Insbesondere kommen beispielsweise die Anionen folgender Säuren in Frage: HBr, HCl, HJ, HF, $HNO_3$, $H_2SO_4$ - ($SO_4^{--}$); $H_3PO_4$ ($HPO_4^{--}$); $H_2CO_3$, ($CO_3^{--}$); HSCN; Kampfersulfonsäure, aliphatische oder aromatische Sulfonsäuren, beispielsweise $C_1$-$C_6$-Alkylsulfonsäuren (zum Beispiel Methansulfonsäure, Ethan-, Propan- oder Hexansulfonsäure), Benzol- oder Naphthalinsulfonsäure, die gegebenenfalls ein- oder zweifach durch Methylgruppen substituiert sind (Toluolsulfonsäure, insbesondere o- oder p-Toluolsulfonsäure), aliphatische $C_1$-$C_{20}$-Monocarbonsäuren insbesondere $C_1$-$C_{18}$-Monocarbonsäuren, die gegebenenfalls ein-, zwei- oder dreifach durch Halogenatome (insbesondere Cl, F) substituiert sind (zum Beispiel Ameisensäure, Essigsäure, Propionsäure, Palmitinsäure, Stearinsäure, Chloressigsäure, Dichloressigsäure, Trifluoressigsäure, Trichloressigsäure); aliphatische $C_2$-$C_{11}$-Dicarbonsäuren, die gegebenenfalls eine Doppelbindung enthalten (zum Beispiel Oxalsäure, Malonsäure, 2-Amino-Malonsäure, Malonsäure, welche in 2-Stellung durch eine Benzylgruppe oder eine oder zwei $C_1$-$C_4$-Alkylgruppen substituiert ist, Maleinsäure, Fumarsäure, Bernsteinsäure); aliphatische Monohydroxy- und Dihydroxy-Monocarbonsäuren mit 2 bis 8, insbesondere 2 bis 6 Kohlenstoffatomen, wobei es sich vorzugsweise um $\alpha$-Monohydroxycarbonsäuren handelt wie Milchsäure, Glycerinsäure oder Glykolsäure; Di- und Tricarbonsäuren mit 3 bis 8 Kohlenstoffatomen, insbesondere 3 bis 6 Kohlenstoffatomen (zum Beispiel Äpfelsäure, Weinsäure, Malonsäure), die auch an einem C-Atom durch eine Hydroxygruppe und/oder gegebenenfalls eine $C_1$-$C_4$-Alkylgruppe substituiert sein können (Isozitronensäure, Zitronensäure); Phthalsäure, die gegebenenfalls durch eine Carboxygruppe (insbesondere in 4-Stellung) substituiert ist; Gluconsäure; Glucuronsäure; Azetidincarbonsäure; Quadratsäure (3,4-Dihydroxy-3-cyclobuten-1,2-dion); die natürlichen $\alpha$-Aminosäuren (zum Beispiel L-Asparaginsäure); 1,1-Cyclobutandicarbonsäure; Organophosphorsäuren, wie Aldose- und Ketosephosphorsäuren (beispielsweise die entsprechenden Mono- und Diphosphorsäuren) zum Beispiel Aldose-6-phosphorsäuren wie D- oder L-Glucose-6-phosphorsäure, $\alpha$-D-Glucose-1-phosphorsäure, D-Fructose-6-phosphorsäure, D-Galactose-6-phosphor-säure, D-Ribose-5-phosphorsäure, D-Fructose-1,6-diphosphor-säuren; Glycerinphosphorsäuren (wobei der Phosphorsäurerest an einem der endständigen oder an dem mittelständigen Glycerinsauerstoffatom gebunden ist) wie $\alpha$-D,L-Glycerin-phosphorsäure, $\beta$-Glycerinphosphorsäure; N-Phosphono-acetyl-Asparaginsäure.

Vorzugsweise bedeutet X jeweils Chlor, Brom, Jod oder -SCN (Rhodanid) oder das Anion X leitet sich von einer Hydroxycarbonsäure der Struktur $R_5$-CH(OH)-$(CH_2)_n$-$CO_2$H ab, wobei n die Werte 0, 1, 2, 3 oder 4 annehmen kann und $R_5$ Wasserstoff, Halogen, Hydroxy, $C_2$-$C_6$-Alkanoyloxy, $C_1$-$C_6$-Alkoxy, $C_1$-$C_6$-Alkyl oder Phenyl, welches gegebenenfalls durch Halogen, Hydroxy, $C_1$-$C_6$-Alkoxy, $C_1$-$C_6$-Alkyl oder $C_1$-$C_6$-Alkanoyloxy substituiert ist, bedeutet.
Im Falle einer solchen Oxycarbonsäure hat der Komplexteil

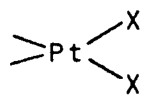

die folgende Struktur

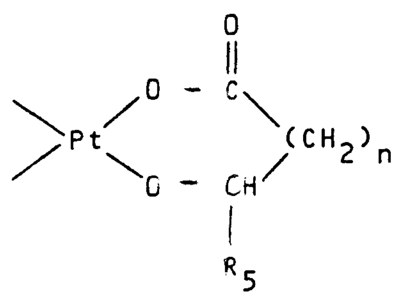

Vorzugsweise leitet sich X von der Milchsäure oder der Glykolsäure (jeweils Racemat, D-Form, L-Form) ab.

Als Säuren für die Anionen X kommen weiterhin in Frage: Aromatische Carbonsäuren, die eine oder mehrere Carboxygruppen enthalten sowie außerdem noch eine oder mehrere (zum Beispiel eine, zwei, drei, vier oder fünf) $C_1$-$C_4$-Alkoxygruppen und/oder Hydroxygruppen. Falls sich mehrere Carboxygruppen an dem aromatischen Rest (zum Beispiel Benzolring) befinden, stehen mindestens 2 Carboxygruppen bevorzugt zueinander in Nachbarstellung. Falls der Benzolring zum Beispiel 4 oder 5 Carboxygruppen enthält, können Komplexe entstehen, die pro 1 Mol des Benzolcarbonsäureanions 2 Mol der Platin-Komponente enthalten. 2 benachbarte Carboxygruppen neutralisieren jeweils 1 Mol der Platinkomponente, so daß zum Beispiel im Falls der Benzolpentacarbonsäure die 1- und 2-ständigen sowie die 4- und 5-ständigen Carboxygruppen jeweils 1 Mol der Platinkomponente absättigen (zusammen also 2 Mol), während die freie 3-ständige Carboxygruppe frei oder in der Salzform mit einem physiologisch verträglichen Kation (zum Beispiel Alkalikation, insbesondere Natriumkation) vorliegt. Dies gilt ganz allgemein, wenn die Anionen X noch zusätzliche Säurefunktionen besitzen, die nicht für die Absättigung des Platins gebraucht werden. Das Analoge gilt für den Fall der Benzolhexacarbonsäure, wobei hier gegebenenfalls 1 Mol dieser Säure 3 Mol der Platinkomponente absättigen kann.

Beispiele für solche Säuren sind: Benzolmonocarbonsäure, Benzoldicarbonsäuren, Benzoltricarbonsäuren (zum Beispiel Trimellithsäure), Benzoltetracarbonsäuren, Benzolpentacarbonsäure, Benzolhexacarbonsäure; Syringasäure, Orotsäure.

Ebenfalls kommen als Säuren, die die Anionen X bilden, Aminosäuren beziehungsweise Aminosäurederivate, deren basische Aminogruppe durch eine Säuregruppe geschützt ist, in Frage. Es handelt sich hierbei zum Beispiel um Aminosäuren der folgenden Struktur:

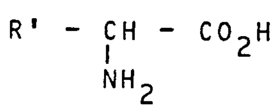

worin R′ Wasserstoff, einen Phenylrest, einen Indolyl-(3)-methylrest,Imidazolyl-(4)-methylrest, eine $C_1$-$C_{10}$-Alkylgruppeoder eine $C_1$-$C_{10}$-Alkylgruppe, die durch eine Hydroxygruppe, eine Carboxygruppe, eine $C_1$-$C_6$-Alkoxygruppe, eine Mercaptogruppe, eine $C_1$-$C_6$-Alkylthiogruppe, eine Phenylgruppe, eine Hydroxyphenylgruppe, eine $C_2$-$C_6$-Alkanoylaminogruppe oder eine $C_1$-$C_6$-Alkoxycarbonylgruppe substituiert ist, bedeutet.

Die basische Aminogruppe in 2-Stellung ist hierbei durch eine übliche Aminosäureschutzgruppe geschützt (acyliert), beispielsweise durch einen $C_2$-$C_6$-Alkanolrest oder den Butyloxycarbonylrest.

Falls in der obigen Formel R′ eine Alkylgruppe ist, handelt es sich vorzugsweise um eine $C_1$-$C_6$-Alkylgruppe,die zum Beispiel in 2-, 3-, 4-, 5- oder 6-Stellung (Zählung beginnt an der Verknüpfungsstelle des Alkylrestes mit dem Restmolekül) eine $C_2$-$C_6$-Alkanoylaminogruppe, einen Imidazolyl-(4)-methylrest oder einen Indolyl-(3)-methylrest enthält. Einzelbeispiele für solche Aminosäuren sind: Leucin (vorzugsweise D- oder L-Form), Valin (vorzugsweise D- oder L-Form), Phenylalanin (vorzugsweise D- oder L-Form), Phenylglycin (vorzugsweise D- oder L-Form), Alanin (vorzugsweise D- oder L-Form), Isoleucin (vorzugsweise D- oder L-Form), Asparagin (vorzugsweise D- oder L-Form), Lysin (vorzugsweise D- oder L-Form), Tryptophan (vorzugsweise D- oder L-Form), Tyrosin (vorzugsweise D- oder L-Form), Ornithin (vorzugsweise D- oder L-Form), Hydroxyprolin (D- oder L-Form).

Hierbei sind die basischen Aminogruppen durch eine übliche Acylaminoschutzgruppe blockiert, insbesondere durch die Acetylgruppe, Chloracetylgruppe oder die Butyloxycarbonylgruppe.

Gegebenenfalls können auch die entsprechenden Säureadditionssalze unter Verwendung physiologisch verträglicher Säuren hergestellt werden, falls die Austauschgruppen X basische Gruppen (zum Beispiel Aminogruppen) enthalten.

Falls X ein Wassermolekül bedeutet, kommen für die Neutralisierung der positiven Ladung des Platinatoms die genannten Säuren in Frage, insbesondere starke Säuren, vorzugsweise $H_2SO_4$.

Die Formel I′ oder I″ umfaßt auch die möglichen Enantiomere und Diastereomere. Falls die Verbindungen Racemate sind, können diese in an sich bekannter Weise, zum Beispiel mittels einer optisch aktiven Säure oder mittels chiraler Phasen in die optisch aktiven Isomere gespalten werden. Es ist aber auch möglich, von vornherein enantiomere oder gegebenenfalls auch diastereomere Ausgangsstoffe einzusetzen, wobei dann als Endprodukt eine entsprechende reine optisch aktive beziehungsweise diastereomere Verbindung erhalten wird. Unabhängig von der Struktur der Reste X besitzt gegebenenfalls auch der Cyclobutanteil asymmetrische Kohlenstoffatome und kann daher in der Racemat-Form oder in einer optisch aktiven beziehungsweise diastereomeren Formen vorliegen.

Zusätzliche Formen entstehen durch Stereochemie am Cyclobutan, wo die beiden Aminomethylgruppen sowie die Reste $R_1$ bis $R_4$ in cis- oder trans-Stellungen stehen können. Weiterhin können zusätliche Formen durch verschiedene enantiomere beziehungsweise diastereomere Formen der Reste X entstehen.

In Bezug auf das Platinatom handelt es sich bei den erfindungsgemäßen Verbindungen der Formel I′ oder I″ insbesondere um die cis-Verbindungen.

Das Ausgangsamin II wird beispielsweise als Racemat, als reine rechts- beziehungsweise linksdrehende Form als cis- oder trans-Form (in Bezug auf die Stellung der Aminomethylgruppen) oder in einer sonstigen diastereomeren Form eingesetzt.

Diese Konfigurationen bleiben bei der Herstellung des Platinkomplexes erhalten.

Das Verfahren zur Herstellung der erfindungsgemäßen Verbindungen I′ wird in einem Lösungsmittel bei Temperaturen zwischen 10 und 80° C, vorzugsweise 20 bis 40° C, insbesondere 25 bis 30° C durchgeführt. Als Lösungsmittel kommen beispielsweise in Frage: Wasser, $C_1$-$C_6$-Alkanole (Methanol, Ethanol, tert.-Butanol), cyclische Ether wie Tetrahydrofuran, Dioxan, gesättigte Ether von ein- oder mehrwertigen Alkoholen wie Ethylenglykoldimethylether, Diethylenglykoldimethylether, niedere gesättigte Ketone (Aceton, Methylethylketon), aprotische Mittel wie Dimethylsulfoxid oder Dialkylamide von niederen aliphatischen Carbonsäuren (Ameisensäure, Essigsäure) mit $C_1$-$C_6$-Alkylresten wie Dimethylformamid, Dimethylacetamid sowie Gemische dieser Lösungsmittel, insbesondere Mischungen mit Wasser.

Die beiden Reaktionskomponenten (Platin-Verbindung und Verbindung II) werden vorzugsweise in äquimolaren Mengen eingesetzt. Der pH-Wert der Reaktionslösung soll zwischen 6 und 9, vorzugsweise bei pH 8 liegen. Die Einstellung des pH-Wertes erfolgt insbesondere durch Zusatz von Alkali, vorzugsweise wäßriger Natronlauge oder Kalilauge oder beispielsweise auch mittels Natriumcarbonat beziehungsweise durch Zusatz von Säuren, vorzugsweise wäßriger Salzsäure. Die Einstellung des pH-Wertes kann auch mittels Ionenaustauschern erfolgen.

Als Tetrahalogen-platin(II)-Verbindungen (Säure sowie Komplexsalze) kommen die entsprechenden Tetrachloro-, Tetrabromo- und Tetrajodo-Verbindungen in Frage. Falls Platin(II)-halogenide als Ausgangskomponente eingesetzt werden, kommen die gleichen Halogenatome in Frage.

Als einwertige Kationen kommen in Betracht: Alkali-Ionen, insbesondere Natrium und Kalium; es können aber auch die Lithium, Rubidium, Cäsium verwendet werden, ebenso $NH_4^+$, $NR_4^+$, $PR_4^+$ oder $AsR_4^+$, in denen R ein $C_1$-$C_6$-Alkylrest oder ein Phenylrest ist. Zweiwertige Kationen können sein: Erdalkali-Ionen, insbesondere $Mg^{2+}$ und $Ca^{2+}$, aber auch $Zn^{2+}$. Als Platin(II)-halogenide kommen beispielsweise $PtCl_2$, $PtBr_2$ und $PtJ_2$ in Frage.

Die Verbindung II wird entweder in Form des Diamins oder in Form eines Säureadditionssalzes eingesetzt: zum Beispiel als Monohydrochlorid oder Dihydrochlorid, Mono- oder Dihydrobromid, Mono- oder Dihydrojodid oder als Satz mit einer anderen üblichen anorganischen oder organischen Säure. Insbesondere kommen auch die Säuren in Frage, deren Anionen die Reste X bilden. Weiterhin kann das Diamin in Form des Acetats beziehungsweise Diacetats eingesetzt werden, wobei gegebenenfalls vor dem Mischen der Reaktionskomponenten Kaliumchlorid (beispielsweise 2 Mol pro 1 Mol Verbindung II) zugesetzt wird. Ebenso kann das Diamin II beispielsweise in Form des Hydrochlorids, Carbonats, Oxalats oder Malonats eingesetzt werden.

Das Verfahren zur Herstellung der Platin(IV)-Komplexe der Formel $I''$ erfolgt beispielsweise in den gleichen Mitteln wie bei dem Verfahren zur Herstellung der Platin(IV)-Komplexe der Formel $I'$. Diese Umsetzungen erfolgen hier in einem Temperaturbereich zwischen 20 und 100° C, vorzugsweise 40 - 80° C. Als Oxidationsmittel kommen in Frage: Halogene wie Chlorgas, Brom, Jod, Wasserstoffperoxid (zum Beispiel 3 bis 60%ig; vorzugsweise 10 bis 40 %, insbesondere 35 %), Dirhodan (gasförmig), Halogenwasserstoffsäuren (HCl, HBr, HJ). Falls die OxYdation mit Halogen, Dirhodan oder Halogenwasserstoffsäuren erfolgt, ist die zusätzliche Anwesenheit einer Verbindung HX gegebenenfalls nicht erforderlich.

Der Austausch der Liganden X gegen andere Liganden kann beispielsweise mittels der Silberhalogenidfällung erfolgen. Hierzu wird beispielsweise eine Dihalogeno-1,2-bis(aminomethyl)cyclobutan-platin(II)-Verbindung der Formel $I'$ oder gegebenenfalls auch eine Verbindung der Formel $I''$, worin X Halogen (Chlor, Brom oder Jod) bedeutet in einem Lösungs- oder Suspensionsmittel bei Temperaturen zwischen 0 bis 90° C, vorzugsweise 10 bis 50° C, insbesondere 30 bis 40° C, vorzugsweise 40° C mit den Silbersalzen einer anderen Säure, die der Bedeutung X entspricht, umgesetzt. Man kann hierbei aber auch als Silbersalz Silbernitrat (zum Beispiel wäßrige Silbernitrat-Lösung) verwenden und erhält (wenn eine Verbindung $I'$ die Ausgangssubstanz ist) einen ionischen Diaquokomplex der Formel

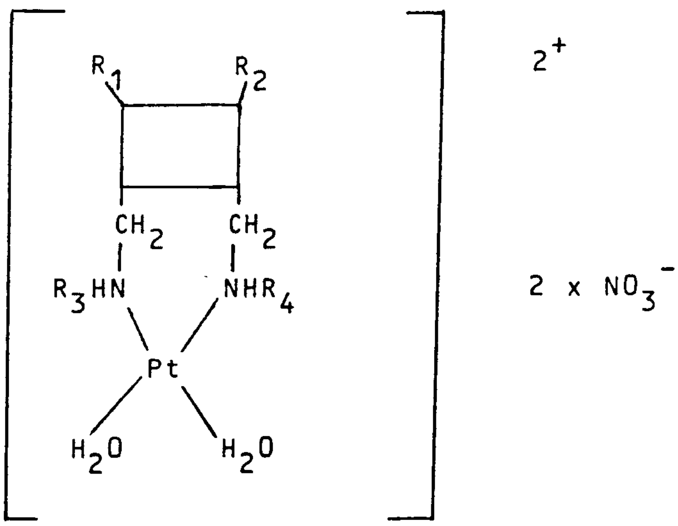

Aus diesem Komplex läßt sich der schwach gebundene Ligand Wasser leicht durch affinere Anionen (zum Beispiel $Cl^-$, $Br^-$ in Form von NaCl, KCl, NaBr, KBr, Malonat$^{2-}$, Chloracetat$^{(-)}$, Oxalat$^{2-}$, 1,1-Cyclobutandicarbonsäure-Anion$^{2-}$ sowie die übrigen angegebenen Säurereste X verdrängen, angewandt in Form der Säuren oder ihrer Salze, insbesondere ihrer Alkalisalze.

Die gleichen Verbindungen lassen sich auch wie folgt gewinnen: Behandeln des zuvor angegebenen Diaquo-Nitratkomplexes mit einem Anionenaustauscher in der Hydroxidform (zum Beispiel Dowex 1 - 8X), wobei die 2 Moleküle Wasser durch OH ersetzt werden und anschließende Umsetzung der so erhaltenen Komplexverbindung (X = jeweils OH) mit der äquimolaren Menge HX wobei X ein physiologisch verträgliches Säureanion ist.

Ein Austausch der Abgangsgruppe (zum Beispiel $SO_4^{2-}$-beziehungsweise Oxalatanion$^{2-}$) ist im Falle der Sulfato- beziehungsweise Oxalato-1,2-bis(aminomethyl)cyclobutan-platin(II)-Verbindungen auch durch Umsetzung mit Erdalkalisalzen, die den gewünschten X-Liganden (zum Beispiel Glycerinsäure) enthalten, möglich, sofern der entstehende Komplex wasserlöslich ist und damit die Abtrennung des schwer wasserlöslichen Erdalkalisulfats oder -oxalats erlaubt.

Für dieses Verfahren geeignete X-Liganden sind vorzugsweise die Anionen von Hydroxycarbonsäuren,

Sulfonsäuren, Halogenessigsäuren, Salpetersäure.

Die Lösungs- beziehungsweise Suspensionsmittel, die für das Herstellungsverfahren der Verbindungen I angegeben wurden, kommen auch für die Austauschreaktion in Frage (insbesondere eignen sich Wasser, Dimethylformamid, Dimethylacetamid, Dimethylsulfoxid, Methanol, Ethanol, tert.-Butanol, Aceton, Methylethylketon). Die Austauschreaktion wird beispielsweise in einem pH-Bereich zwischen 3 und 9 durchgeführt.

Die Herstellung von unbekannten Ausgangsaminen der Formel II kann beispielsweise wie bei Beispiel 1 beschrieben ist, aus den entsprechenden bekannten Cyclobutan-1,2-dicarbonsäuren (mit den Substituenten $R_1$ und $R_2$ in 3-Stellung sowie $R_3$ und $R_4$ in 4-Stellung) erfolgen.

Die im Cyclobutanring durch die Reste $R_1$, $R_2$, $R_3$ und $R_4$ substituierte Cyclobutan-1,2-dicarbonsäure wird über das entsprechende Säuredihalogenid (Chlorid, Bromid) mittels Ammoniak oder einem Amin $NHR_5$ beziehungsweise $NHR_6$ in bekannter Weise zum entsprechenden Amid umgesetzt und dieses dann in hierfür bekannter Weise durch Hydrierung (zum Beispiel katalytische Hydrierung oder mittels komplexen Hydriden wie $LiAlH_4$) zum Diamin der Formel II reduziert.

Weiterhin ist die Herstellung von solchen Ausgangsaminen nach folgenden Methoden möglich: Katalytische Hydrierung von entsprechenden Dicyan-Verbindungen in Gegenwart von hierfür üblichen Metallkatalysatoren gemäß der Britischen Patentschrift 1 121 413; Reduktion mit Lithiumaluminiumhydrid in Diethylether beziehungsweise Tetrahydrofuran; Überführung in die entsprechenden Säureamide mittels Ameisensäure/HCl und anschließende Reduktion mit Lithiumaluminiumhydrid in Tetrahydrofuran; Curtiusschen Abbau der entsprechenden Säureazide; Abbau nach der Methode von K. F. Schmidt (siehe zum Beispiel J. Am. Soc. 64 (1942) Seiten 269-98).

Die Amino-Substituenten $R_5$ und/oder $R_6$ werden eingeführt, indem man die Synthese von Diaminen II mit den entsprechenden Aminosubstituenten beginnt.

Zur weiteren Erläuterung wird auf die Herstellung einiger Ausgangsverbindungen der Formel II bei den Beispielen verwiesen.

Die erfindungsgemäßen Verbindungen zeigen beispielsweise an der P388-Leukämie der Maus eine gute Antitumorwirkung.

Beispielsweise wird bei oben genannter Versuchsmethode bei einer intraperitonealen Dosis von 10 - 30 mg/kg Körpergewicht Maus eine Überlebenszeitverlängerung von 77 % erzielt. Die niedrigste bereits wirksame Dosis in dem oben angegebenen Tierversuch ist beispielsweise

| | |
|---|---|
| 2 mg/kg | oral |
| 0,5 mg/kg | intraperitoneal |
| 0,5 mg/kg | intravenös |

Als allgemeiner Dosisbereich für die Wirkung (Tierversuch wie oben) kommt beispielsweise in Frage:

| | |
|---|---|
| 2 - 2000 mg/kg | oral, insbesondere 50 - 200 mg/kg |
| 0,5 - 1000 mg/kg | intraperitoneal, insbesondere 2 - 100 mg/kg |
| 0,5 - 1000 mg/kg | intravenös, insbesondere 2 - 100 mg/kg |

Die Wirkungsrichtung der erfindungsgemäßen Verbindungen ist mit der Wirkung des bekannten Arzneimittelwirkstoffs Cis-Platin vergleichbar, jedoch bestehen hierzu insbesondere folgende Unterschiede: bessere Wirksamkeit, anderes Wirkspektrum, nahezu keine Nephrotoxizität.

Indikationen für die die erfindungsgemäßen Verbindungen in Betracht kommen können: Chemotherapie bösartiger Erkrankungen (Krebs).

Die pharmazeutichen Zubereitungen enthalten im allgemeinen zwischen 1 bis 2000 vorzugsweise 10 bis 1000 mg der erfindungsgemäßen aktiven Komponente(n).

Die Verabreichung kann beispielsweise in Form von Tabletten, Kapseln, Pillen, Dragees, Zäpfchen, Salben, Gelees, Cremes, Puder, Stäubepulver, Aerosolen oder in flüssiger Form erfolgen. Als flüssige Anwendungsformen kommen zum Beispiel in Frage: Ölige oder alkoholische beziehungsweise wäßrige Lösungen sowie Suspensionen und Emulsionen. Bevorzugte Anwendungsformen sind Tabletten, die zwischen 100 und 1000 mg oder Lyophilisate (beispielsweise zur Herstellung von Lösungen), die zwischen 10 und 200 mg an aktiver Substanz enthalten.

Die Einzeldosis der erfindungsgemäßen aktiven Komponenten kann beispielsweise liegen

a) bei oralen Arzneiformen zwischen 10 und 2000 mg, vorzugsweise 10 bis 1000 mg;

b) bei parenteralen Arzneiformen (zum Beispiel intravenös, intramuskulär) zwischen 1 und 1000, vorzugsweise 5 bis 200 mg.

Beispielsweise können 3mal täglich 1 bis 4 Tabletten mit einem Gehalt von 10 bis 500 mg wirksamer Substanz oder zum Beispiel bei intravenöser Injektion 1 bis 4 mal täglich eine Ampulle mit 1 bis 200 mg Substanz empfohlen werden. Bei oraler Verabreichung ist die minimale tägliche Dosis beispielsweise 1 mg; die maximale tägliche Dosis bel oraler Verabreichung soll nicht über 2000 mg liegen.

Für die Behandlung von Hunden und Katzen liegt die orale Einzeldosis im allgemeinen zwischen ungefähr 10 und 500 mg/kg Körpergewicht; die parenterale Dosis ungefähr zwischen 1 und 500 mg/kg Körpergewicht.

Für die Behandlung von Pferden und Vieh liegt die orale Einzeldosis im allgemeinen zwischen ungefähr 10 und 500 mg/kg; die parenterale Einzeldosis ungefähr zwischen 1 und 500 mg/kg Körpergewicht.

Die akute Toxizität der erfindungsgemäßen Verbindungen an der Maus (ausgedrückt durch die LD 50 mg/kg; Methode nach Miller und Tainter: Proc. Soc. Exper. Biol. a. Med. 57 (1944) 261) liegt beispielsweise bei intraperitonealer Applikation zwischen 5 und 1000 mg/kg.

Die Arzneimittel können in der Humanmedizin, der Veterinärmedizin sowie in der Landwirtschaft allein oder im Gemisch mit anderen pharmakologischen aktiven Stoffen verwendet werden.

Die erfindungsgemäßen Verbindungen sind zur Herstellung pharmazeutischer Zusammensetzungen geeignet. Die pharmazeutischen Zusammensetzungen beziehungsweise Medikamente können eine oder mehrere der erfindungsgemäßen Verbindungen oder auch Mischungen derselben mit anderen pharmazeutisch wirksamen Stoffen enthalten. Zur Herstellung der pharmazeutischen Zubereitungen können die üblichen pharmazeutischen Träger- und Hilfsstoffe verwendet werden. Die Arzneimittel können zum Beispiel enteral, parenteral (zum Beispiel intravenös, intramuskulär, intraperitoneal, subkutan) oder oral angewendet werden. Beispielsweise kann die Verabreichung in Form von Tabletten, Kapseln, Pillen, Dragees oder Zäpfchen erfolgen. Als Liquida kommen zum Beispiel in Frage: ölige oder wäßrige Lösungen oder Suspensionen (zum Beispiel in Sesam- oder Olivenöl), Emulsionen, injizierbare wäßrige und ölige Lösungen oder Suspensionen. Weiterhin können beispielsweise Trockenampullen, welche als Wirkstoff die erfindungsgemäße Verbindung I enthalten, hergestellt werden, wobei vor Gebrauch der Inhalt solcher Trockenampullen zum Beispiel in physiologischer Kochsalzlösung oder Gemischen aus physiologischer Kochsalzlösung und beispielsweise Dimethylsulfoxid aufgelöst wird.

Beispiel 1

Dichloro-1,2-bis(aminomethyl)cyclobutan-Platin(II) (trans-Form)

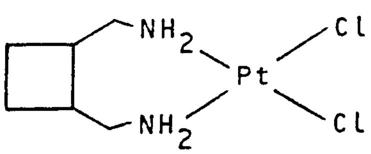

Zu einer Lösung von 3,05 g (0,0073 mol) Kaliumtetrachloroplatinat in 10 ml Wasser werden 0,81 g (0,014 mol) KOH und 1,5 g (0,0073 mol) 1,2-Bis(aminomethyl)cyclobutan bei einer Temperatur von 50° C zugegeben und 3 Stunden rühren lassen. Nach dem Abkühlen auf Raumtemperatur wird abgesaugt, mit Wasser und mit Aceton/Diethylether 1:1 gewaschen. Ausbeute: 1,0 g
F.: 225 - 226° C (Zersetzung)

Herstellung des Ausgangsamins II

A) 5 g (0,028 mol) trans-Cyclobutan-1,2-dicarbonsäuredichlorid werden zu einer Mischung von 50 ml konzentrierter Ammoniaklösung und 50 ml Eis zugetropft. Nach der Zugabe wird noch eine Stunde gerührt, dann wird der Niederschlag (Säureamid) abgesaugt, mit Wasser gewaschen und aus 150 ml Ethanol umkristallisiert (Ausbeute: 2,5 g).
F.: 231 - 233° C

Das so erhaltene Säureamid wird nun mit Lithiumaluminiumhydrid zum Diamin II reduziert:

8 g (0,21 mol) Lithiumaluminiumhydrid werden in Stickstoffatmosphäre in 200 ml wasserfreiem Tetrahydrofuran suspendiert. Bei einer Temperatur von 0° C werden 5 g (0,035 mol) Amid portionsweise vorsichtig eingetragen. Nach Ende der Zugabe wird noch eine Stunde bei Raumtemperatur gerührt und 4

Stunden unter Rückfluß erhitzt. Nach Stehen über Nacht wird zunächst Essigester, dann Wasser zugegeben und vom Niederschlag durch Filtration getrennt. Das Filtrat wird über Magnesiumsulfat getrocknet und am Rotationsverdampfer eingeengt. Der Rückstand wird mit Isopropylalkohol aufgenommen und das Salz mit 7 g (0,07 mol) Oxalsäure gefällt und aus Ethanol umkristallisiert (Ausbeute: 4,7 g). Das Dioxalat schmilzt bei 160° C unter Zersetzung.

B) 115 g (3 mol) $LiAlH_4$ werden bei -10°C in 1500 ml Diethylether mit einer Lösung von 53 g (0,5 mol) 1,2-Dicyanocyclobutan (in 500 ml Diethylether gelöst) versetzt. Man läßt über Nacht stehen und hydrolysiert mit 185 ml Essigester und 350 ml Wasser. Der Niederschlag wird abgesaugt, mit Ether gewaschen und das Filtrat zur Trockene am Rotationsverdampfer eingeengt.

Man erhält 38 g 1,2-Bis(aminomethyl)cyclobutan, welches in 550 ml Ethanol gelöst, mit 50,4 g Oxalsäure versetzt wird. Der Niederschlag wird abgesaugt und mit wenig Ether gewaschen. Man erhält 68 g Dioxalat. F. 160°C (Zersetzung)

Darstellung des Lactato-Komplexes (Beispiel 1a)

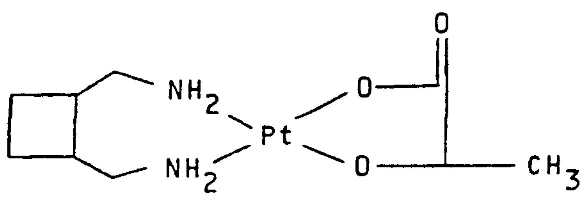

3,8 g (0,01 mol) Chloro-Komplex werden in 20 ml Wasser suspendiert und auf 40° C erwärmt. 3,39 g (0,02 mol) Silbernitrat werden zugegeben und 1,5 Stunden rühren lassen. Nach dem Abkühlen der Mischung im Kühlschrank wird vom Silberchlorid-Niederschlag abgesaugt und mit 10 ml Wasser gewaschen. Das Filtrat wird über eine Säule mit 100 ml eines basischen Ionenaustauschers perkoliert und in 1 g (0,01 mol) L-Milchsäure eingetropft. Nach 3tägigem Rühren bei Raumtemperatur wird eingeengt, der Rückstand in Methanol gelöst und unter Zusatz von Aktivkohle gerührt. Nach Filtration wird mit Diethylether bis zur Trübung versetzt und im Kühlschrank über Nacht kristallisieren lassen. Die auf der Fritte nach dem Absaugen gesammelten Kristalle werden mit Diethylether gewaschen und bei 40° C getrocknet. Ausbeute: 1,2 g
F.: 220° C (Zersetzung)

Beispiele für weitere Komplexe des 1,2-Bis(aminomethyl)cyclobutan-platin(II) mit verschiedenem Anion X

Allgemeine Herstellungsmethode:

3,8 g (0,01 Mol) Dichloro-1,2-bis(aminomethyl)cyclobutan-platin(II) werden in 20 ml Wasser, dem 1 ml Ethanol zugesetzt ist, suspendiert und mit 3,39 g (0,02 Mol) $AgNO_3$ versetzt. Nach dem Erwärmen auf 40° C wird ca. 5 Stunden bei der angegebenen Temperatur gerührt. Nach dem Abkühlen auf 15° C (Kühlschrank) wird vom ausgefallenen AgCl abfiltriert und der Rückstand mit 10 ml Wasser gewaschen. Das Filtrat läßt man nach tropfenweiser Perkolation über eine Säule mit 100 ml basischen Ionenaustauscher (OH-Typ) dann in eine Lösung von 0,01 Mol der neuen Abgangsgruppe (X) in 5 ml Wasser zulaufen. Die so erhaltene Mischung wird über Nacht gerührt, sodann eingeengt und mittels Säulenchromatographie aus Aceton/Wasser an Silicagel gereinigt.

Die erhaltenen Komplexe sind in der Tabelle 1 aufgeführt:

Tabelle 1

| Strukturteil 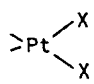 und zugrunde liegende Säure | Ausbeute | |
|---|---|---|
| b) 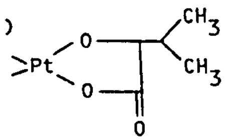 D,L-2-Hydroxy-3-methylbuttersäure | 1,9 g | weißes Pulver<br>Rf-Wert: 0,44<br>Silicagel Aceton/Wasser 4 : 1 |
| c) 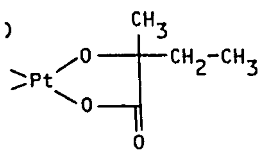 2-Hydroxy-2-methylbuttersäure | 1,95 g | weißes Pulver<br>Schmelzpunkt 202-203°C |
| d) 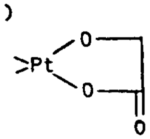 Glycolsäure | 1,6 g | weißes Pulver<br>Schmelzpunkt 250-251°C |
| e) 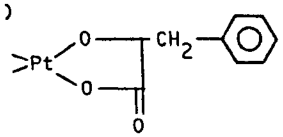 L(-)-3-Phenylmilchsäure | 2,5 g | Schmelzpunkt 195-196°C |

| Strukturteil 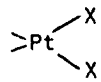 und zugrunde liegende Säure | Ausbeute | |
|---|---|---|
| f) 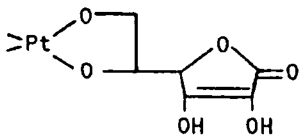 L-Ascorbinsäure | 2,0 g | Schmelzpunkt 203-210°C |
| g) 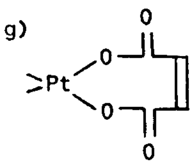 Maleinsäure | 2,9 g | Rf-Wert: 0,33<br>Silicagel Aceton/Wasser 4 : 1 |
| h) 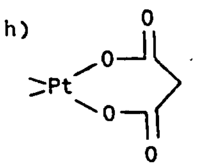 Malonsäure | 2,5 g | Rf-Wert: 0,41<br>Silicagel Propanol/Wasser 5 : 1 |
| i) 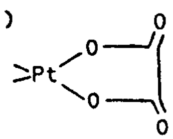 Oxalsäure | 2,6 g | Rf-Wert: 0,38<br>Silicagel Aceton/Wasser 4 : 1 |

| Strukturteil 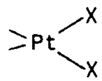 und zugrunde liegende Säure | Ausbeute | |
|---|---|---|
| j) 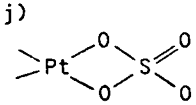 Schwefelsäure | 2,8 g | Schmelzpunkt 280-284°C |
| k) 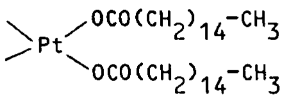 Palmitinsäure | 3,2 g | Schmelzpunkt 75-80°C |
| l) 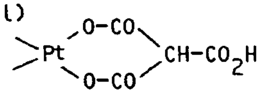 1,2,3-Propantricarbonsäure | 2,4 g | Schmelzpunkt 253-255°C |
| m) 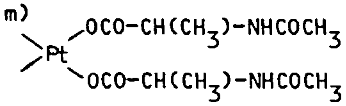 N-Acetyl-alanin | 2,15 g | Schmelzpunkt 88-92°C |

| Strukturteil 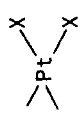 und zugrunde liegende Säure | Ausbeute | |
|---|---|---|
| n) 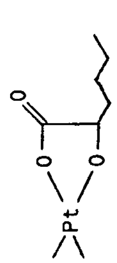 D,L-2-Hydroxyhexansäure | 2,2 g | Schmelzpunkt 187-189°C |
| o) 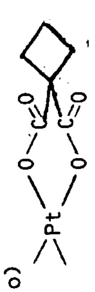 Cyclobutan-1,1-dicarbonsäure | 1,8 g | Schmelzpunkt 240-245°C |

Die Herstellung des Komplexes gemäß m) erfolgt hierbei gemäß folgender Vorschrift:
2,9 g Dichloro-1,2-bis(aminomethyl)cyclobutan-platin(II) werden in 50 ml Wasser suspendiert und mit 2,6 Silbernitrat versetzt. Man rührt 4 Stunden bei 50°C, saugt dann den Silberchlorid-Niederschlag ab, setzt den farblosen Filtrat 2 g N-acetyl-alanin und 0,85 g KOH zu und rührt weitere 5 Stunden bei 30°C. Die Reaktionsmischung wird gefriergetrocknet und der Rückstand aus Ethanol umkristallisiert.

Beispiel 2

Dichloro-1,2-bis(methylamino-methyl)cyclobutan-platin(II)

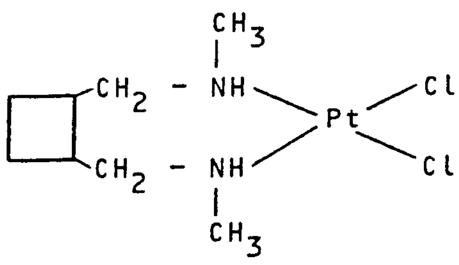

1,79 g (0,0043 mol) Kaliumtetrachloroplatinat II werden in 5 ml Wasser bei 50° C gelöst und mit 0,5 g (0,0086 mol) KOH und 1 g (0,0043 mol) 1,2-Bis-(methylamino-methyl)cyclobutan versetzt. Nach zweistündigem Rühren wird auf Raumtemperatur gekühlt und abgesaugt.
Ausbeute.: 0,53 g

Das Ausgangsamin kann z. B. wie folgt erhalten werden:
10 g Cyclobutan-1,2-dicarbonsäuredichlorid werden unter Eiskühlung in 200 ml gesättigte Methylamin-Lösung eingetropft. Nach 4 Stunden Rühren bei Raumtemperatur wird am Rotationsverdampfer eingeengt und der Rückstand aus 900 ml Essigester umkristallisiert.
Man erhält 5,6 g Cyclobutan-1,2-dicarbonsäure-methylamid.
(F. 180 - 181° C).
Zu 7,06 g (0,186 mol) $LiAlH_4$ in 173 ml Tetrahydrofuran (gekühlt in einem Eisbad) werden in kleinen Portionen 5,3 g (0,031 mol) des so erhaltenen Amids zugegeben. Nach einstündigem Rühren und weiterer Zugabe von 93 ml Tetrahydrofuran wird unter Rückfluß gekocht. Nach Stehen über Nacht wird unter Eiskühlung zunächst mit Essigester zersetzt, sodann Wasser zugegeben. Nach einstündigem Rühren wird filtriert und über $K_2CO_3$ getrocknet und am Rotationsverdampfer eingeengt. Der Rückstand wird mit Ethanol aufgenommen und mit 6,5 g Oxalsäure (gelöst in 20 ml Wasser) versetzt. Das ausgefallene Produkt wird einmal aus Ethanol umkristallisiert. Das Oxalat (2 Mol Oxalsäure auf ein Mol Amin) schmilzt bei 145 - 147° C (1,3 g).

Beispiel 3 (Pt(IV)-Komplex)

Dichloro-dihydroxy-[1,2-Bis(aminomethyl)cyclobutan]-Platin(IV)

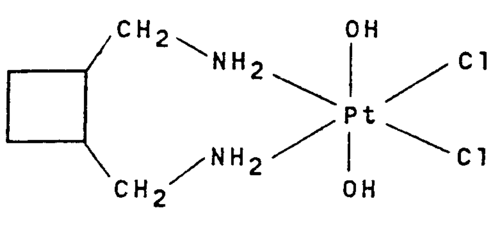

0,5 g (0,0013 mol) Dichloro-1,2-bis(aminomethyl)cyclobutan-platin(II) werden in 10 ml Wasser bei 70°C suspendiert und tropfenweise mit 5 ml $H_2O_2$ (35%ige Lösung) versetzt. Es wird 4 Stunden bei 70°C gerührt (pH 4) und über Nacht kühl gestellt. Der orange-gelbe Niederschlag wird abgesaugt und mit wenig Wasser gewaschen. Das Filtrat wird zur Entfernung des nicht verbrauchten Wasserstoffperoxids mit 200 mg Platin-Aktivkohle versetzt und 3 Stunden bei Raumtemperatur gerührt, die Platin-Aktivkohle sodann abgesaugt. Das Filtrat wird zur Trockene eingeengt.
Ausbeute: 200 mg gelbes Pulver

Beispiel 4 (Pt(IV)-Komplex)

Tetrachloro-[1,2-Bis(aminomethyl)cyclobutan]-Platin(IV)

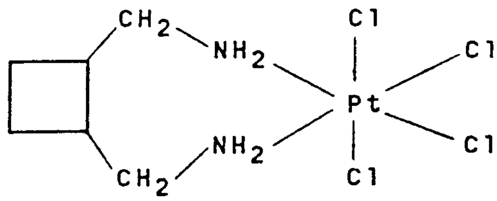

2 g (0,0053 mol) Dichloro-1,2-bis(aminomethyl)cyclobutan-platin(II) werden in 250 ml Wasser suspendiert und bei Raumtemperatur mit Chlorgas umgesetzt. Es wird 4 Stunden Chlorgas eingeleitet; dabei entsteht eine Lösung, aus welcher im weiteren Verlauf der Reaktion ein orange-gelbes Produkt ausfällt, das abgesaugt, mit Wasser gewaschen und bei 40 °C im Vakuum getrocknet wird.
Ausbeute 0,85 g

Beispiel 5 (Pt(IV)-Komplex)

Dihydroxy-lactato-[1,2-Bis(aminomethyl)cyclobutan]-Platin(IV)

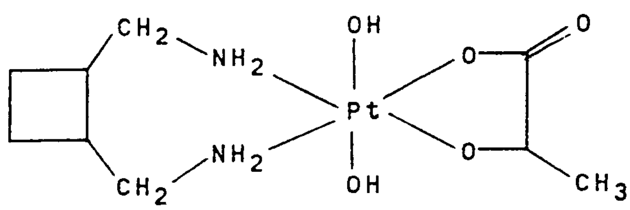

0,5 g Lactato-[1,2-bis(aminomethyl)cyclobutan]-platin(II) werden in 10 ml Wasser bei 70 °C gelöst und mit 5 ml $H_2O_2$ Lösung (35%ig) versetzt. Nach 4 Stunden rühren bei 70 °C (pH 5) wird über Nacht abgekühlt. Das überschüssige $H_2O_2$ wird mit Platin-Aktivkohle zersetzt, die Aktivkohle abgesaugt und das Filtrat zur Trockene eingeengt
Ausbeute: 200 mg gelbes Pulver

Beispiele für pharmazeutische Zubereitungen

Beispiel für Kapseln

1 kg Lactato-Komplex gemäß Beispiel 1a, 625 g mikrokristalline Cellulose und 11 g hochdisperses Siliciumdioxid werden durch ein Sieb der Maschenweite 0,8 mm gegeben und homogenisert. Anschließend werden dieser Mischung 39 g Magnesiumstearat (gesiebt 0,8 mm), hinzugefügt und nochmals 1 Minute gemischt.
Zur Herstellung der Kapseln wird die Kapselmasse in bekannter Weise auf einer mit Formatteilen der Größe 00 ausgerüsteten Kapselmaschine in Hartgelatinekapseln der Größe 00 abgefüllt. Die Füllmenge pro Kapsel beträgt 670 mg entsprechend 400 mg Wirkstoff.

Beispiel für Lyophilisat

In 900 ml Wasser für Injektionszwecke werden 20 g Lactato-Komplex gemäß Beispiel 1a unter Rühren gelöst. Anschließend wird mit Wasser für Injektionszwecke auf 1 Liter aufgefüllt.
Diese Lösung wird unter aseptischen Bedingungen über ein Membranfilter von 0,22 µm Porenweite sterilfiltriert und zu 2 ml in 10 ml Injektionsflaschen der hydrolytischen Klasse I abgefüllt. Die Flaschen werden mit einem Gefriertrocknungsstopfen versehen und in einer geeigneten Anlage lyophilisiert. Nach der Trocknung begast man mit sterilem, getrocknetem Stickstoff und verschließt die Flaschen in der Anlage. Die Stopfen werden durch eine Bördelkappe gesichert. Für die intravenöse Anwendung wird das Lyophilisat in 4 ml Wasser für Injektionszwecke aufgelöst.

1 Injektionsflasche enthält 40 mg Verbindung gemäß Beispiel 1a, 1 ml Lösung enthält 10 mg Wirkstoff.

**Patentansprüche**

1. 1,2-Bis(aminomethyl)cyclobutan-Platin-Komplexe der allgemeinen Formel

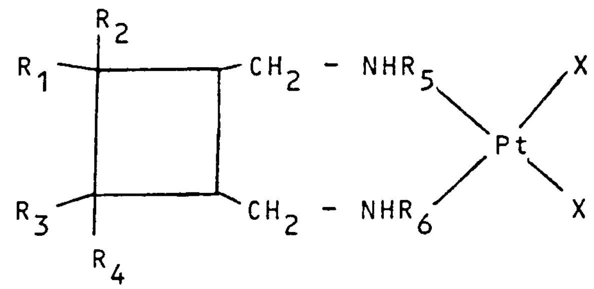

I' oder

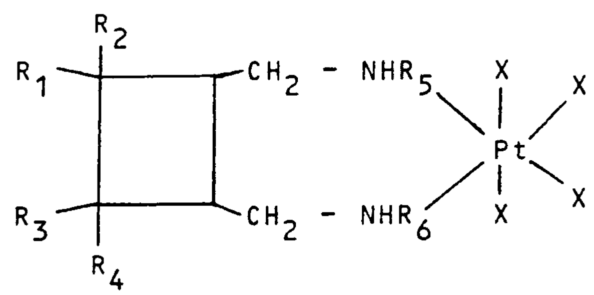

I''

worin die Reste $R_1$, $R_2$, $R_3$, $R_4$, $R_5$ und $R_6$ gleich oder verschieden sind und Wasserstoff, $C_1$-$C_6$-Alkyl, Phenyl, Phenyl-$C_1$-$C_6$-alkyl, $C_1$-$C_6$-Alkyl, welches durch Halogen, Hydroxy, $C_2$-$C_6$-Alkanoyloxy oder $C_1$-$C_6$-Alkoxy substituiert ist, Phenyl, welches durch Halogen, Hydroxy, $C_2$-$C_6$-Alkanoyloxy oder $C_1$-$C_6$-Alkoxy substituiert ist oder Phenyl-$C_1$-$C_6$-alkyl, welches im Phenylteil durch Halogen, Hydroxy, $C_2$-$C_6$-Alkanoyloxyoder $C_1$-$C_6$-Alkoxy substituiert ist, bedeuten und X für das Äquivalent eines physiologisch verträglichen Anions steht oder X auch ein Wassermolekül bedeuten kann und in diesem Falle die vorhandene positive Ladung des Platinatoms ebenfalls durch ein physiologisch verträgliches Anion abgesättigt wird, und deren Salze.

2. Verfahren zur Herstellung von 1,2-Bis(aminomethyl)cyclobutan-Platin(II)-Komplexen der allgemeinen Formel

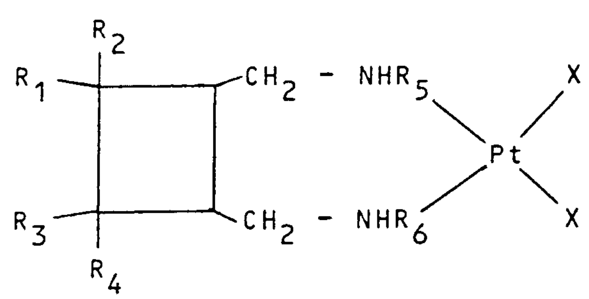

I'

worin die Reste $R_1$, $R_2$, $R_3$, $R_4$, $R_5$ und $R_6$ gleich oder verschieden sind und Wasserstoff, $C_1$-$C_6$-Alkyl, Phenyl, Phenyl-$C_1$-$C_6$-alkyl, $C_1$-$C_6$-Alkyl, welches durch Halogen, Hydroxy, $C_2$-$C_6$-Alkanoyloxy oder $C_1$-$C_6$-Alkoxy substituiert ist, Phenyl, welches durch Halogen, Hydroxy, $C_2$-$C_6$-Alkanoyloxy oder $C_1$-$C_6$-Alkoxy substituiert ist oder Phenyl-$C_1$-$C_6$-alkyl, welches im Phenylteil durch Halogen, Hydroxy, $C_2$-$C_6$-

Alkanoyloxyoder $C_1$-$C_6$-Alkoxy substituiert ist, bedeuten und X für das Äquivalent eines physiologisch verträglichen Anions steht oder X auch ein Wassermolekül bedeuteten kann und in diesem Falle die vorhandene positive Ladung des Platinatoms ebenfalls durch ein physiologisch verträgliches Anion abgesättigt wird,
sowie gegebenenfalls deren Salzen,
dadurch gekennzeichnet,
daß man eine Tetrahalogeno-platin(II)-säure,
ein Tetrahalogeno-platin(II)-Komplexsalz mit zwei einwertigen oder einem zweiwertigen Kation oder ein Platin(II)-halogenid mit einer Verbindung der Formel

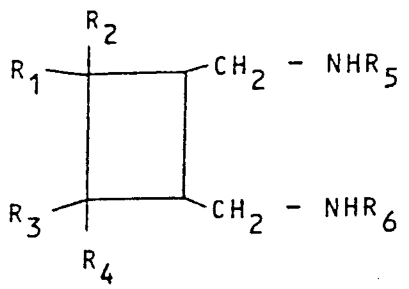

II

oder einem Salz der Verbindung II mit einem physiologisch verträglichen Gegenion
oder einem Säureadditionssalz der Verbindung II umsetzt, wobei die Reste $R_1$ bis $R_6$ die angegebenen Bedeutungen haben
und gegebenenfalls in einer erhaltenen Verbindung der Formel I den Rest X beziehungsweise die Reste X gegen andere physiologisch verträgliche Anionen austauscht und/oder gegebenenfalls erhaltene Verbindungen in die Salze mit physiologisch verträglichen Anionen oder Kationen überführt.

3. Verfahren zur Herstellung von 1,2-Bis(aminomethyl)cyclobutan-Platin(IV)-Komplexen der allgemeinen Formel

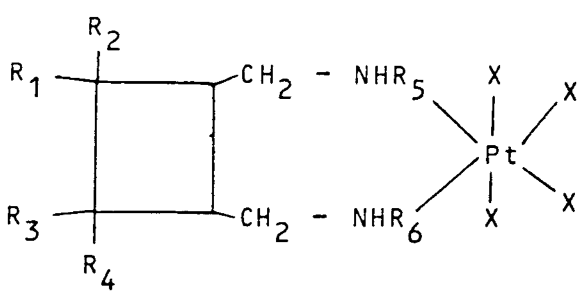

I''

worin die Reste $R_1$, $R_2$, $R_3$, $R_4$, $R_5$ und $R_6$ gleich oder verschieden sind und Wasserstoff, $C_1$-$C_6$-Alkyl, Phenyl, Phenyl-$C_1$-$C_6$-alkyl, $C_1$-$C_6$-Alkyl, welches durch Halogen, Hydroxy, $C_2$-$C_6$-Alkanoyloxy oder $C_1$-$C_6$-Alkoxy substituiert ist, Phenyl, welches durch Halogen, Hydroxy, $C_2$-$C_6$-Alkanoyloxy oder $C_1$-$C_6$-Alkoxy substituiert ist oder Phenyl-$C_1$-$C_6$-alkyl, welches im Phenylteil durch Halogen, Hydroxy, $C_2$-$C_6$-Alkanoyloxyoder $C_1$-$C_6$-Alkoxy substituiert ist, bedeuten und X für das Äquivalent eines physiologisch verträglichen Anions steht oder X auch ein Wassermolekül bedeuteten kann und in diesem Falle die vorhandene positive Ladung des Platinatoms ebenfalls durch ein physiologisch verträgliches Anion abgesättigt wird,
sowie gegebenenfalls deren Salzen,
dadurch gekennzeichnet, daß man eine Platin-Komplex-Verbindung der Formel I′ worin die Reste $R_1$ bis $R_6$ sowie X die angegebenen Bedeutungen haben oxydiert, gegebenenfalls in Gegenwart einer Verbindung HX und gegebenenfalls bei dem so erhaltenen Reaktionsprodukt einen oder mehrere der Reste X gegen andere physiologisch verträgliche Anionen austauscht und/oder gegebenenfalls erhaltene Verbindungen in die Salze mit physiologisch verträglichen Anionen oder Kationen überführt.

4. Arzneimittel, enthaltend eine Verbindung der allgemeinen Formel I′ oder I″ neben üblichen Träger- und/oder Verdünnungs- beziehungsweise Hilfsstoffen.

5. Verfahren zur Herstellung eines Arzneimittels, dadurch gekennzeichnet, daß eine Verbindung der allgemeinen Formel I′ oder I″ mit gebräuchlichen pharmazeutischen Trägerstoffen oder Verdünnungsmitteln beziehungsweise sonstigen Hilfsstoffen zu pharmazeutischen Zubereitungen verarbeitet beziehungsweise in eine therapeutisch anwendbare Form gebracht wird.

6. Verwendung von Verbindungen der allgemeinen Formel I′ oder I″ zur Herstellung von Arzneimitteln.

**Claims**

1. 1,2-bis-(aminomethyl)-cyclobutane/platinum complexes corresponding to the general formula

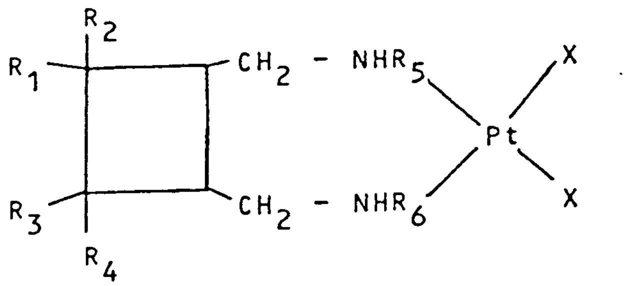

I' or

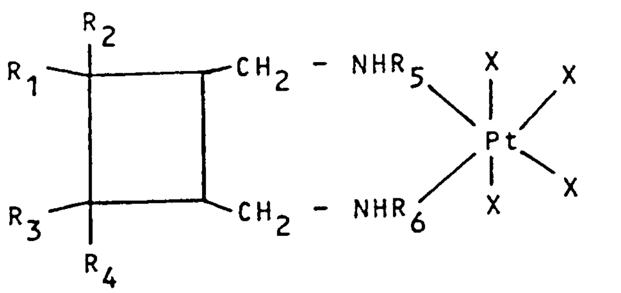

I''

in which the substituents $R_1$, $R_2$, $R_3$, $R_4$, $R_5$ and $R_6$ may be the same or different and represent hydrogen, $C_{1-6}$ alkyl, phenyl, phenyl-$C_{1-6}$-alkyl, $C_{1-6}$ alkyl substituted by halogen, hydroxy, $C_{2-6}$ alkanoyloxy or $C_{1-6}$ alkoxy, phenyl substituted by halogen, hydroxy, $C_{2-6}$ alkanoyloxy or $C_{1-6}$ alkoxy or phenyl-$C_{1-6}$-alkyl substituted in the phenyl moiety by halogen, hydroxy, $C_{2-6}$ alkanoyloxy or $C_{1-6}$ alkoxy and X is the equivalent of a physiologically safe anion or X may even be a water molecule, in which case the positive charge of the platinum atom present is also saturated by a physiologically safe anion,
and salts thereof.

2. A process for the preparation of 1,2-bis-(aminomethyl)-cyclobutane/platinum (II) complexes corresponding to the following general formula:

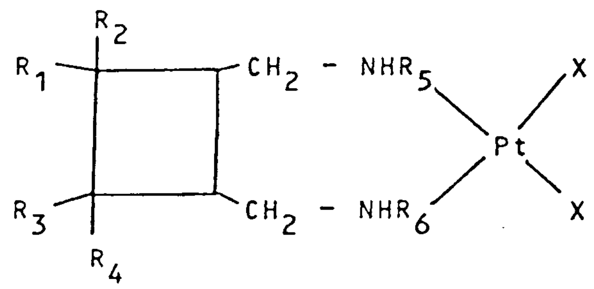

I'

in which the substituents $R_1$, $R_2$, $R_3$, $R_4$, $R_5$ and $R_6$ may be the same or different and represent hydrogen, $C_{1-6}$ alkyl, phenyl, phenyl-$C_{1-6}$-alkyl, $C_{1-6}$ alkyl substituted by halogen, hydroxy, $C_{2-6}$ alkanoyloxy or $C_{1-6}$ alkoxy, phenyl substituted by halogen, hydroxy, $C_{2-6}$ alkanoyloxy or $C_{1-6}$ alkoxy or phenyl-$C_{1-6}$-alkyl substituted in the phenyl moiety by halogen, hydroxy, $C_{2-6}$ alkanoyloxy or $C_{1-6}$ alkoxy and X is the equivalent of a physiologically safe anion or X may even be a water molecule, in which case the positive charge of the platinum atom present is also saturated by a physiologically safe anion,
and optionally salts thereof,
characterized in that a tetrahydroplatinic (II) acid, a tetrahaloplatinum (II) complex salt containing two monovalent cations or one divalent cation or a platinum (II) halide is reacted with a compound corresponding to the formula

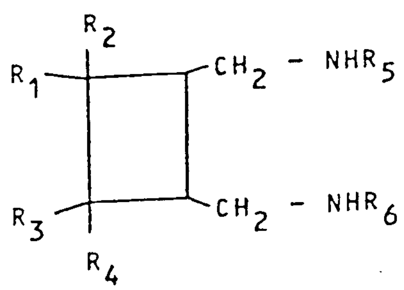

II

in which the substituents $R_1$ to $R_6$ are as defined above, or with a salt of compound II with a physiologically safe counterion or with an acid addition salt of compound II and, in the resulting compound of formula I, the substituent X or the substituents X is/are optionally replaced by other physiologically safe anions and/or the compounds obtained are optionally converted into the salts with physiologically safe anions or cations.

3. A process for the preparation of 1,2-bis-(aminomethyl)-cyclobutane/platinum (IV) complexes corresponding to the general formula

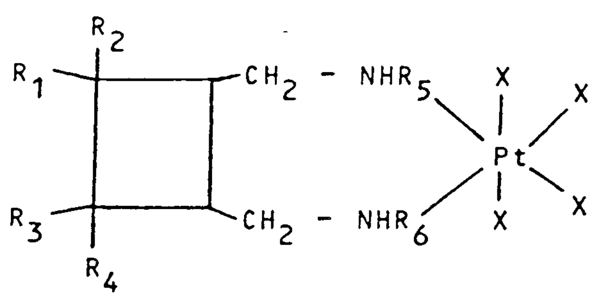

I''

in which the substituents $R_1$, $R_2$, $R_3$, $R_4$, $R_5$ and $R_6$ may be the same or different and represent hydrogen, $C_{1-6}$ alkyl, phenyl, phenyl-$C_{1-6}$-alkyl, $C_{1-6}$ alkyl substituted by halogen, hydroxy, $C_{2-6}$ alkanoyloxy or $C_{1-6}$ alkoxy, phenyl substituted by halogen, hydroxy, $C_{2-6}$ alkanoyloxy or $C_{1-6}$ alkoxy or phenyl-$C_{1-6}$-alkyl substituted in the phenyl moiety by halogen, hydroxy, $C_{2-6}$ alkanoyloxy or $C_{1-6}$

alkoxy and X is the equivalent of a physiologically safe anion or X may even be a water molecule, in which case the positive charge of the platinum atom present is also saturated by a physiologically safe anion,
and optionally salts thereof,
characterized in that a platinum complex compound corresponding to formula I', in which the substituents $R_1$ to $R_6$ and X are as defined above, are oxidized, optionally in the presence of a compound HX, and one or more of the substituents X in the reaction product obtained are optionally replaced by other physiologically safe anions and/or the compounds obtained are optionally converted into the salts with physiologically safe anions or cations.

**4.** A medicament containing a compound corresponding to general formula I' or I'' in addition to typical excipients and/or diluents or auxiliaries.

**5.** A process for the preparation of a medicament, characterized in that a compound corresponding to general formula I' or I'' is processed with typical pharmaceutical excipients or diluents or other auxiliaries to form pharmaceutical preparations or is brought into a therapeutically useable form.

**6.** The use of compounds corresponding to general formula I' or I'' for the preparation of medicaments.

## Revendications

**1.** Complexes de platine et de 1,2-bis-(aminométhyl)cyclobutane de formule générale

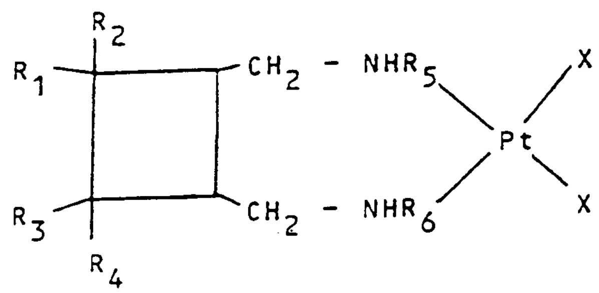

I' ou

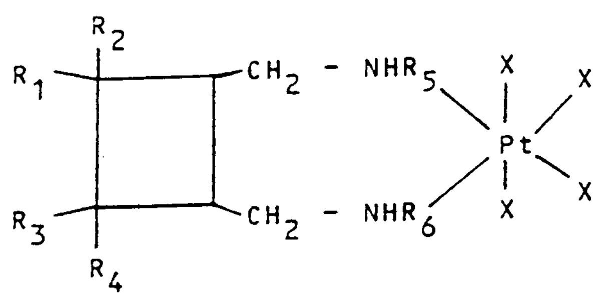

I''

dans laquelle les restes $R_1$, $R_2$, $R_3$, $R_4$, $R_5$ et $R_6$ sont identiques ou différents et représentent des atomes d'hydrogène, des restes alkyle en $C_1$-$C_6$, phényle, phénylalkyle en $C_1$-$C_6$, alkyle en $C_1$-$C_6$ qui sont substitues par des atomes d'halogène ou des groupements hydroxy, alcanoyloxy en $C_2$-$C_6$ ou alcoxy en $C_1$-$C_6$, phényle qui sont substitués par des atomes d'halogène ou des groupements hydroxy, alcanoyloxy en $C_2$-$C_6$ ou alcoxy en $C_1$-$C_6$, ou phénylalkyle en $C_1$-$C_6$ qui sont substitués, dans la partie phényle, par des atomes d'halogène ou des groupements hydroxy, alcanoyloxy en $C_2$-$C_6$ ou alcoxy en $C_1$-$C_6$, et X est mis pour l'équivalent d'un anion acceptable physiologiquement, ou X peut aussi représenter une molécule d'eau et, dans ce cas, la charge positive existante de l'atome de platine est également saturée par un anion acceptable physiologiquement, et sels de ces complexes.

**2.** Procédé de préparation de complexes de platine(II) et de 1,2-bis-(aminométhyl)-cyclobutane de formule générale

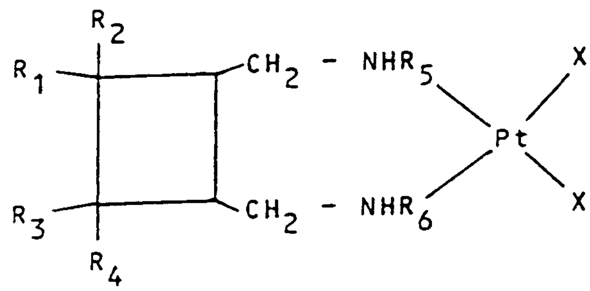

I'

dans laquelle les restes $R_1$, $R_2$, $R_3$, $R_4$, $R_5$ et $R_6$ sont identiques ou différents et représentent des atomes d'hydrogène, des restes alkyle en $C_1$-$C_6$, phényle, phénylalkyle en $C_1$-$C_6$, alkyle en $C_1$-$C_6$ qui sont substitués par des atomes d'halogène ou des groupements hydroxy, alcanoyloxy en $C_2$-$C_6$ ou alcoxy en $C_1$-$C_6$, phényle qui sont substitués par des atomes d'halogène ou des groupements hydroxy, alcanoyloxy en $C_2$-$C_6$ ou alcoxy en $C_1$-$C_6$, ou phénylalkyle en $C_1$-$C_6$ qui sont substitués, dans la partie phényle, par des atomes d'halogène ou des groupements hydroxy, alcanoyloxy en $C_2$-$C_6$ ou alcoxy en $C_1$-$C_6$, et X est mis pour l'équivalent d'un anion acceptable physiologiquement, ou X peut aussi représenter une molécule d'eau et, dans ce cas, la charge positive existante de l'atome de platine est également saturée par un anion acceptable physiologiquement, et éventuellement de sels de ces complexes, caractérisé en ce qu'on fait réagir un acide tétrabalogénoplatineux, un sel complexe de tétrahalogénoplatine(II) avec deux cations monovalents ou un cation bivalent ou un halogénure de platine(II), avec un composé de formule

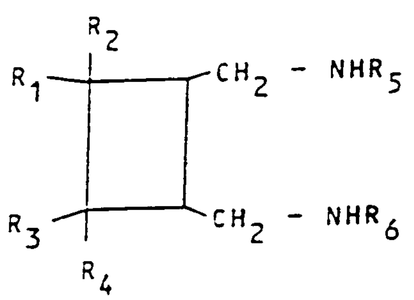

II

ou un sel du composé II avec un contre-ion acceptable physiologiquement ou un sel d'addition d'acide du composé II, les restes $R_1$ à $R_6$ ayant les significations sus-indiquées, on échange éventuellement, dans le composé de formule I obtenu, le reste X ou les restes X contre d'autres anions acceptables physiologiquement et/ou on transforme éventuellement les composés obtenus en leurs sels avec des anions ou des cations acceptables physiologiquement.

**3.** Procédé de préparation de complexes de platine(IV) et de 1,2-bis-(aminométhyl)-cyclobutane de formule générale

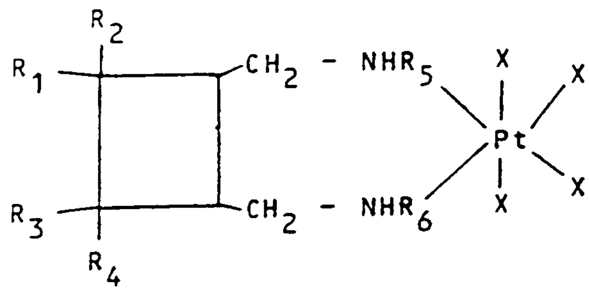

I''

dans laquelle les restes $R_1$, $R_2$, $R_3$, $R_4$, $R_5$ et $R_6$ sont identiques ou différents et représentent des atomes d'hydrogène, des restes alkyle en $C_1$-$C_6$, phényle, phénylalkyle en $C_1$-$C_6$, alkyle en $C_1$-$C_6$ qui sont substitués par des atomes d'halogène ou des groupements hydroxy, alcanoyloxy en $C_2$-$C_6$ ou alcoxy en $C_1$-$C_6$, phényle qui sont substitués par des atomes d'halogène ou des groupements hydroxy, alcanoyloxy en $C_2$-$C_6$ ou alcoxy en $C_1$-$C_6$, ou phénylalkyle en $C_1$-$C_6$ qui sont substitués, dans la partie phényle, par des atomes d'halogène ou des groupements hydroxy, alcanoyloxy en $C_2$-$C_6$ ou alcoxy en $C_1$-$C_6$, et X est mis pour l'équivalent d'un anion acceptable physiologiquement, ou X peut aussi représenter une molécule d'eau et, dans ce cas, la charge positive existante de l'atome de platine est également saturée par un anion acceptable physiologiquement, et éventuellement de sels de ces complexes, caractérisé en ce qu'on oxyde un composé complexe du platine de formule I', dans laquelle les restes $R_1$ à $R_6$ ainsi que X ont les significations susindiquées, éventuellement en présence d'un composé HX, on échange éventuellement, dans le produit réactionnel obtenu, un ou plusieurs des restes X contre d'autres anions acceptables physiologiquement et/ou on transforme éventuellement les composés obtenus en leurs sels avec des anions ou des cations acceptables physiologiquement.

**4.** Médicament, contenant un composé de formule générale I' ou I'', outre des excipients et/ou des diluants ou adjuvants usuels.

**5.** Procédé de préparation d'un médicament, caractérisé en ce qu'on travaille un composé de formule générale I' ou I'' avec des excipients ou des diluants ou autres adjuvants pharmaceutiques usuels pour former des préparations pharmaceutiques ou on les met sous une forme applicable en thérapeutique.

**6.** Utilisation de composés de formule générale I' ou I'' pour la préparation de médicaments.